# FASCICULE DE BREVET EUROPEEN

(11) **EP 3 236 843 B1**
(45) Date de publication et mention de la délivrance du brevet: **17.02.2021**
(21) Numéro de dépôt: 15821083.1
(22) Date de dépôt: 23.12.2015
(51) Int. Cl.: A61B 5/046, A61B 5/04, A61B 5/00

(54) **CARTOGRAPHIE RÉGIONALE HAUTE DENSITÉ DU SUBSTRAT DE LA FIBRILLATION ATRIALE**
HOCHDICHTE REGIONALE ABBILDUNG DES VORHOFFLIMMERSUBSTRATS
REGIONAL HIGH-DENSITY MAPPING OF THE ATRIAL FIBRILLATION SUBSTRATE

(30) Priorité: 23.12.2014 FR 1463232
(43) Date de publication de la demande: 01.11.2017
(73) Titulaire: SUBSTRATE HD, 13008 Marseille (FR)
(72) Inventeur: BARS, Clément, 92100 Boulogne Billancourt (FR); SEITZ, Julien, 13008 Marseille (FR)
(74) Mandataire: Cabinet Netter
(86) Numéro de dépôt international: PCT/EP2015/081193
(87) Numéro de publication internationale: WO 2016/102685

(56) Documents cités:
- WO-A1-2005/115232
- US-A1- 2012 232 417
- DRS SEITZ ET AL: "Automated Detection of Complex Fractionated AtrialElectrograms in Substrate-Based Atrial Fibrillation Ablation: Better Discrimination with a New Setting of CARTO Algorithm", JOURNAL FOR ATRIAL FIBRILLATION : JAFIB, vol. 6, no. 2, 1 janvier 2013 (2013-01-01) , pages 16-23, XP055220049, ISSN: 1941-6911
- SCHERR ET AL: "Automated detection and characterization of complex fractionated atrial electrograms in human left atrium during atrial fibrillation", HEART RHYTHM, ELSEVIER, US, vol. 4, no. 8, 2 août 2007 (2007-08-02), pages 1013-1020, XP022183931, ISSN: 1547-5271, DOI: 10.1016/J.HRTHM.2007.04.021
- NADEMANEE KOONLAWEE ET AL: "A new approach for catheter ablation of atrial fibrillation: mapping of the electrophysiologic substrate", JOURNAL OF THE AMERICAN COLLEGE OF CARDIOLOGY, ELSEVIER, NEW YORK, NY, US, vol. 43, no. 11, 2 juin 2004 (2004-06-02), pages 2044-2053, XP002578066, ISSN: 0735-1097, DOI: 10.1016/J.JACC.2003.12.054 cité dans la demande
- KWABENA A BOAHENE ET AL: "Termination of acute atrial fibrillation in the Wolff-Parkinson-White syndrome by procainamide and propafenone: Importance of atrial fibrillatory cycle length *", JOURNAL OF THE AMERICAN COLLEGE OF CARDIOLOGY, ELSEVIER, NEW YORK, NY, US, vol. 16, no. 6, 1 novembre 1990 (1990-11-01), pages 1408-1414, XP008177782, ISSN: 0735-1097, DOI: 10.1016/0735-1097(90)90384-2

## Description

### Domaine technique

La présente invention concerne le domaine technique de l'identification de zones du cœur susceptibles d'être impliquées dans une arythmie cardiaque. Plus particulièrement, la présente invention concerne un procédé et un appareil pour l'identification de zones du cœur susceptibles d'être impliquées dans l'initiation et la perpétuation de la fibrillation atriale (FA).

### État de la technique

La fibrillation atriale (FA), ou parfois appelée communément « arythmie », est le trouble du rythme cardiaque le plus fréquent et le plus complexe.

Ce trouble du rythme cardiaque se manifeste par une activité atriale très rapide et désorganisée se traduisant par une fréquence cardiaque rapide et irrégulière.

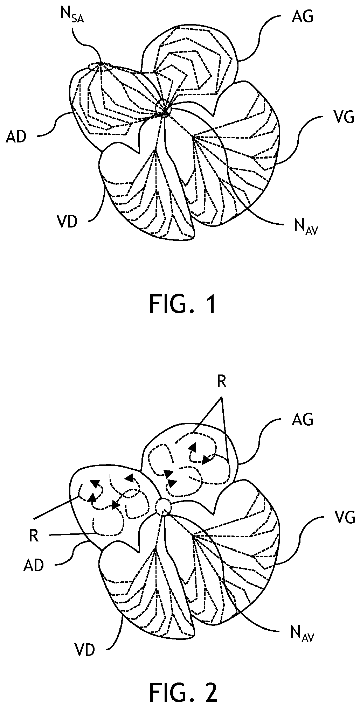

Dans un cœur qui ne présente pas d'arythmie à un instant donné (voir figure 1), la contraction cardiaque résulte d'un processus de dépolarisation (inversion de la polarité électrique de la membrane des cellules qui entraine leur activation afin qu'elles remplissent leur fonction, dans le cas du cœur, la contraction) cyclique d'un groupe (**N_{SA}**) de cellules situé au niveau de la partie haute de l'oreillette droite (**OD**) constituant le nœud sinusal (ou encore appelé nœud sino-atrial). Cette dépolarisation se propage rapidement à la manière d'une vague de manière ordonnée de haut en bas et de proche en proche à l'ensemble des cellules des deux oreillettes droite et gauche **OD, OG,** ce qui déclenche leur contraction quasi synchrone. La propagation se fait ensuite vers le nœud atrio-ventriculaire (**N_{AV}**) et puis vers les deux ventricules droite et gauche **VD, VG,** ce qui provoque alors leur contraction de manière légèrement retardée par rapport à celle des oreillettes. La propagation de la dépolarisation est ordonnée, et peut être représentée par des rayons partant du nœud sinusal qui se divisent à leur tour et ainsi de suite puis convergent jusqu'au nœud atrio-ventriculaire duquel partent d'autres rayons qui se divisent également et ainsi de suite (voir les lignes en pointillés sur la figure 1).

Lorsqu'il y a fibrillation atriale (voir figure 2), la propagation de la dépolarisation n'est plus ordonnée et la dépolarisation se propage au sein des oreillettes **OD, OG** de façon très rapide et désorganisée.

Les mécanismes initiateurs de la FA sont maintenant bien connus ; il s'agit le plus souvent d'extrasystoles provenant généralement d'une ou de plusieurs des veines pulmonaires (dans l'oreillette gauche). Par contre, les mécanismes responsables de la perpétuation de la FA sont mal connus (substrat arythmogène).

Plusieurs théories existent, mais une des plus plausibles est la théorie des rétro-boucles **R** (*rotors* en anglais). Il s'agit d'une propagation de la dépolarisation qui suit un chemin en forme de boucle, on parle de circuits de réentrée (en anglais *reentries*)*.*Ces rétro-boucles peuvent être plus ou moins nombreuses et peuvent s'activer de façon alternée.

La fibrillation atriale peut également être due à des sources focales (appelés en anglais *focal sources*)*,* c'est-à-dire des zones où les cellules sont hyperexcitables conduisant à une fréquence d'excitation très rapide.

Ces rétro-boucles et sources focales ont été récemment documentées chez l'homme : Narayan et al.,« Treatment of atrial fibrillation by the ablation of localized sources: CONFIRM (Conventional ablation for atrial fibrillation with or without focal impulse and rotor modulation) trial », (en français : « Traitement de la fibrillation atriale par l'ablation de sources localisées : CONFIRM (ablation conventionnelle pour la fibrillation atriale avec ou sans modulation d'impulsion focales ou de rotor) »), in J.Am. Coll. Cardiol., 60, 628-636 (2012) et Haissaguerre et al., « Driver domains in persistent atrial fibrillation » (en français : « Zones motrices dans la fibrillation atriale persistante »), in Circulation, 12 août 2014 ;130(7):530-8.

Afin de soigner la FA, les zones soupçonnées de contribuer à ce mécanisme anormal sont ablatées (le tissu des zones soupçonnées est détruit, le plus souvent par radiofréquence, mais tout autre source d'énergie peut être utilisée). Plusieurs techniques d'ablation du substrat de la FA ont été proposées et aucun consensus n'existe aujourd'hui.

Par ailleurs, différentes approches pour identifier les tissus soupçonnés d'être responsables de la fibrillation ont été mises au point pour guider l'ablation.

Une première approche pour identifier les tissus soupçonnés d'être responsables de la FA est la détection de potentiels fragmentés complexes (*complexfractionated atrial electrogramsen* anglais, ou encore sous l'acronyme CFAE) a été développée par le Dr Nademanee en 2004: K. Nademanee et al., «A new approach for catheter ablation of atrial fibrillation: mapping of the electrophysiologicsubstrate» (en français «Une nouvelle approche pour l'ablation par cathéter dans la fibrillation atriale : cartographie du substrat électrophysiologique »), in J. Am. Coll. Cardiol. 2 juin 2004 ; 43(11) : 2044-53.

On suspecte aujourd'hui fortement que les CFAE correspondent aux signaux électriques enregistrés sur les sites de ces rétro-boucles ou de ces sources focales. Cette approche permet d'arrêter la FA dans 99% des cas et 95% des cas selon les publications : K. Nademanee, *ibidem* ; J. Seitz et al., « Active or passive pulmonaryvein in atrial fibrillation: is pulmonary vein isolation always essential? » (en français : « Veine pulmonaire active ou passive dans la fibrillation atriale : l'isolement de veines pulmonaires est-il toujours essentiel ? »), in Heart Rhythm, avril 2014 ; 11(4) : 579-86. La technique développée par le Dr Nademanee est également celle qui donne les meilleurs résultats (publiés) immédiats et à long terme.

Cependant, de nombreux CFAE sont dits passifs et leur ablation est inutile et/ou inefficace, voire délétère. Seule l'analyse visuelle d'opérateurs experts permet à ce jour de discriminer les CFAE actifs des passifs. Malheureusement, l'analyse visuelle des CFAE conduit à une faible reproductibilité car elle est difficile pour des opérateurs non experts.

Pour cette raison, des logiciels de détection automatique des CFAE ont été développés (Biosense Webster et Saint Jude Medical). Ces logiciels analysent les électrogrammes (EGMs) zone par zone dans les oreillettes. Pendant la durée d'analyse par zone (2,5 sec pour le logiciel de Biosense Webster), les durées (généralement en ms) entre les déflexions par rapport à la ligne de base (électrogrammes) sont mesurées. Plusieurs algorithmes peuvent alors être mis en œuvre et les résultats affichés à l'aide d'un code couleur afin de guider les praticiens :
- l'algorithme SCI (pour *Shortest Complex Interval* en anglais, soit intervalle complexe le plus court) dans lequel pour chaque zone, la couleur affichée correspond à la valeur de la durée du plus petit intervalle entre deux CFAE enregistré pendant la durée d'analyse ;
- l'algorithme ICL (pour *Interval Confidence Level* en anglais, soit niveau de confiance de l'intervalle) dans lequel pour chaque zone, la couleur affichée correspond au nombre d'intervalles entre deux CFAE enregistrés pendant la durée d'analyse (voire par exemple le document "Automated detection and characterization of complex fractionated atrial electrograms in human left atrium during atrial fibrillation", Scherr et al, HEART RHYTHM, ELSEVIER, US, vol. 4, no. 8, 2 août 2007 (2007-08-02), pages 1013-1020) à ; et
- l'algorithme ACI (pour *Average Complex Interval* en anglais, soit intervalle complexe moyen) dans lequel pour chaque zone, la couleur affichée correspond à la valeur moyenne de la durée de l'ensemble des intervalles entre deux CFAE enregistrés pendant la durée d'analyse.

Malheureusement, ces logiciels présentent nombre d'inconvénients dont les suivants.
- Ils utilisent la définition du terme CFAE donnée par le Dr Nademanee qui ne définit pas de manière suffisamment précise les potentiels électriques fractionnés intéressants qui reflètent le maintien de l'arythmie, ce qui conduit à un nombre élevé de faux positifs, et *in fine* à un nombre élevé d'ablations inutiles. En effet avec cette analyse seule la mesure de la durée entre deux déflexions électriques (évènements) (encore appelée communément « cycles ») est prise en compte, sans différencier la durée des électrogrammes et des lignes de bases.
- Les seuils de détections des intervalles cibles sont définies de manière absolue sans prendre en compte la fréquence (ou le cycle) de la fibrillation atriale qui est propre à chaque patient. La fréquence de la fibrillation atriale a une influence directe sur la fragmentation des potentiels électriques atriaux (plus la fréquence est rapide et plus les potentiels se fragmentent) : Konings et al.,« Configuration of unipolar atrial electrograms during electrically induced atrial fibrillation in humans » (en français : « Configuration d'électrogrammes atriaux unipolaires pendant la fibrillation atriale induite électriquement chez l'Homme »),in Circulation,1997;95 :1231-1241.
- Ils ne tiennent pas compte de l'existence ou de l'absence d'une répétition des événements mesurés, d'un battement à un autre.
- Ils ne tiennent pas compte, dans l'analyse des électrogrammes, des potentiels dont l'amplitude est supérieure à un seuil donné. Tenir compte du voltage (Comme le fait Biosense Webster) est important, mais l'élimination d'une déflexion de haute amplitude dans l'analyse des cycles peut être problématique. En effet, il est possible parfois d'avoir des potentiels d'amplitude supérieur au seuil donné qui soient intéressants pour l'analyse de l'électrogramme.

Ainsi, ces procédés automatisés qui auraient pu aider un plus grand nombre de praticiens à identifier les zones du cœur à ablater (substrat arythmogène de la FA) sont aujourd'hui encore insuffisamment performants.

Une autre technique de cartographie a été développée par Narayan et al. (« Treatment of atrial fibrillation by the ablation of localized sources: CONFIRM (Conventional ablation for atrial fibrillation with or without focal impulse and rotor modulation) trial, inJ.Am. Coll. Cardiol., 60, 628-636 (2012)). Celle-ci a pour but de détecter les rotors directement. La méthode consiste en une analyse simultanée dans chacune des oreillettes par de sondes multi-électrodes de grande taille (64 électrodes par oreillettes) insérées à l'aide de cathéters et permettant une analyse globale ou « panoramique » des deux oreillettes (et non pas régionales) afin de détecter les rétro-boucles et les sources focales.

Les résultats obtenus grâce à ces cartes de localisation de rotors et de sources focales sont cependant décevants. En effet, l'article susmentionné indique un taux de 52 % de retour en rythme normal (aussi appelé rythme sinusal).

Les faibles résultats de cette méthode sont éventuellement liés à l'absence de précision dans cette cartographie (vision globale mais peu précise, l'absence de prise en compte de la morphologie des potentiels électriques (ou électrogrammes) et la difficulté pour reconstruire la propagation électrique en cas de potentiels fragmentés.

Une approche plus récente présentée à deux congrès internationaux en 2014 (Seitz et Bars, Heart Rhythm Society 2014 à San Francisco & ISCAT 2014 à Paris) consiste à réaliser une cartographie visuelle régionale à l'aide d'une sonde multi-électrodes. Cette approche permet une analyse de la fragmentation et de l'activation afin de détecter les rétro-boucles et leur conduction lente sous-jacentes. Cette technique donne de très bons résultats avec 96% d'arrêt de FA et un bon taux de reproductibilité (7 opérateurs).

Malheureusement, l'analyse selon cette méthode est une analyse visuelle dont les résultats dépendent de l'opérateur.

### Présentation de l'invention

Un objectif de l'invention est donc de pallier au moins un des inconvénients de la technique antérieure mentionnés ci-dessus.

En particulier, un objectif de l'invention est de proposer un procédé pour identifier les zones du cœur susceptibles d'être impliquées dans la perpétuation de la fibrillation atriale (FA) de manière plus fiable que ce qui est aujourd'hui possible, afin d'aider le praticien dans son choix de traitement.

Pour cela, l'invention propose un procédé mis en oeuvre par un processeur pour l'identification de zones du cœur d'un patient susceptibles d'être impliquées dans la perpétuation de la fibrillation atriale, ci-après zones cibles, à partir d'une pluralité d'enregistrements locaux réalisés sur le cœur du patient pendant une fenêtre de mesure, chaque enregistrement local correspondant à une zone du cœur du patient, chaque enregistrement local comprenant au moins un électrogramme local et éventuellement au moins une ligne de base, le procédé comprenant les étapes suivantes :
- l'obtention d'un cycle de référence de l'arythmie, le cycle de référence étant la durée moyenne des lignes de base entre deux électrogrammes locaux d'un enregistrement local correspondant à une zone saine du cœur du patient ; et
- pour chaque enregistrement local :
   ▪ la détermination de lignes de base entre deux électrogrammes locaux ;
   ▪ le calcul d'au moins un ratio parmi :
      ∘ un ratio moyen de la durée de la ligne de base sur le cycle de référence de l'arythmie ;
      ∘ un ratio médian de la durée de la ligne de base sur le cycle de référence de l'arythmie ;
      ∘ pour chaque ligne de base, un ratio de la durée de la ligne de base sur le cycle de référence de l'arythmie ;
   ▪ la classification de la zone du cœur correspondante selon son degré d'implication dans la perpétuation de la FA, le degré d'implication de la zone étant fonction du ou des ratios calculés.

Cette invention permet une analyse fine des signaux électriques afin de déterminer les sources ciblées par l'ablation par le biais d'une quantification du pourcentage de cycle de référence de la FA enregistrée dans différentes régions des oreillettes. La prise en compte du cycle de référence de la FA permet de s'affranchir de nombres des problèmes décrits ci-dessus.

En outre, une zone peut être classée comme une zone non-cible si le ratio moyen, le ratio médian, ou tous les ratios calculés pour les lignes de base de l'enregistrement local correspondant est/sont supérieur(s) ou égal/égaux à une première valeur, la zone étant classée comme une zone cible vrai ou faux positif sinon.

En outre ou alternativement, une zone peut classée comme une zone cible prioritaire vrai ou faux positif si le ratio moyen, le ratio médian, ou au moins un ratio calculé pour une ligne de base de l'enregistrement local correspondant est inférieur ou égal à une deuxième valeur, le cas échéant inférieure à la première valeur.

En outre ou alternativement, une zone peut classée comme une zone cible secondaire vrai ou faux positif sile ratio moyen, le ratio médian, ou au moins un ratio calculé pour une ligne de base de l'enregistrement local correspondant est compris entre la deuxième valeur et une troisième valeur comprise entre les première et deuxième valeurs ; et
classée comme une zone cible tertiaire vrai ou faux positif si le ratio moyen, le ratio médian, ou au moins un ratio calculé pour une ligne de base de l'enregistrement local correspondant est compris entre la troisième valeur et la première valeur.

En outre ou alternativement, la classification de chacune des zones du cœur peut être réalisée selon l'une des modalités suivantes :
- elle est également fonction de la répétition au sein de l'enregistrement local correspondant des lignes de base à ratio calculé de valeurs répétitives ;une zone cible étant un vrai positif si la répétition correspondant à la valeur de ratio ayant conduit à la classification de la zone comme étant une zone cible est avérée ;
- elle est également fonction de la moyenne des valeurs d'amplitudes des dépolarisations de l'enregistrement local correspondant; une zone cible étant un vrai positif si la moyenne des valeurs d'amplitudes est inférieure à une valeur seuil donnée ;
- elle est également fonction de la répétition au sein de l'enregistrement local correspondant des lignes de base à ratio calculé de valeurs répétitives et fonction de la moyenne des valeurs d'amplitudes des dépolarisations de l'enregistrement local correspondant ;une zone cible étant un vrai positif si la répétition est avérée et si la moyenne des valeurs d'amplitudes est supérieure à une valeur seuil donnée.

En outre ou alternativement, le procédé peut comprendre pour au moins une partie des enregistrements locaux du cœur :
- l'obtention d'au moins un enregistrement régional à partir d'une pluralité d'enregistrement locaux correspondant à des zones adjacentes, les zones adjacentes formant une région correspondant à l'enregistrement régional, les enregistrements locaux ne présentant aucune ligne de base identifiable étant écartés, ainsi l'enregistrement régional comprend au moins un électrogramme régional ; et
- pour chaque enregistrement régional :
   ▪ la détermination de lignes de base entre deux électrogrammes régionaux ;
   ▪ le calcul d'au moins un ratio parmi :
      ∘ un ratio moyen de la durée de la ligne de base sur le cycle de référence de l'arythmie ; et
      ∘ pour chaque ligne de base, un ratio de la durée de la ligne de base sur le cycle de référence de l'arythmie ;
   ▪ la classification de la région du cœur correspondante selon son degré d'implication dans la perpétuation de la FA, le degré d'implication de la région étant fonction du ou des ratios calculés ;
   les autres étapes du procédés étant réalisées en remplaçant :
- enregistrement local par enregistrement régional et
- zone par la région.

Cette dernière modalité correspond à une analyse multipiste, c'est-à-dire que l'analyse se fait concrètement sur plusieurs enregistrements (chacun étant enregistré sur une piste) en même temps.

Dans un autre aspect de l'invention, celle-ci propose la réalisation de l'analyse multipiste sans passer par l'analyse monopiste, mais toujours en prenant en compte le ratio. Ceci se traduit par un procédé pour l'identification de régions du cœur d'un patient susceptibles d'être impliquées dans la perpétuation de la fibrillation atriale, ci-après régions cibles, à partir d'une pluralité d'enregistrements régionaux réalisés sur le cœur du patient pendant une fenêtre de mesure, chaque enregistrement régional correspondant à une région du cœur du patient regroupant une pluralité de zones, le procédé comprenant les étapes suivantes :
- l'obtention d'un cycle de référence de l'arythmie, le cycle de référence étant la durée moyenne des lignes de base entre deux dépolarisations d'un enregistrement local correspondant à une zone saine du cœur du patient ; et
- pour chaque enregistrement régional :
   ▪ la détermination de lignes de base entre les dépolarisations des enregistrements régionaux durant la fenêtre de mesure ;
   ▪ le calcul d'au moins un ratio parmi :
      ∘ un ratio moyen de la durée de la ligne de base sur le cycle de référence de l'arythmie ;
      ∘ pour chaque ligne de base, un ratio de la durée de la ligne de base sur le cycle de référence de l'arythmie ;
   ▪ la classification de la région du cœur correspondante selon son degré d'implication dans la perpétuation de la FA, le degré d'implication de la région étant fonction du ou des ratios calculés.

En outre, une région peut être classée comme une région non-cible si le ratio moyen ou si tous les ratios calculés pour les lignes de base de l'enregistrement régional correspondant est/sont supérieur(s) ou égal/égaux à une première valeur, la région étant classé comme une région cible vrai ou faux positif sinon.

En outre ou alternativement, une région peut être classée comme une région cible prioritaire vrai ou faux positif si le ratio moyen ou au moins un ratio calculé pour une ligne de base de l'enregistrement régional correspondant est inférieur ou égal à une deuxième valeur, le cas échéant inférieure à la première valeur.

En outre ou alternativement, une région peut être classée comme une région cible secondaire vrai ou faux positif si le ratio moyen ou au moins un ratio calculé pour une ligne de base de l'enregistrement régional correspondant est compris entre la deuxième valeur et une troisième valeur comprise entre les première et deuxième valeurs ; et
comme une région cible tertiaire vrai ou faux positif si le ratio moyen ou au moins un ratio calculé pour une ligne de base de l'enregistrement régional correspondant est compris entre la troisième valeur et la première valeur.

En outre ou alternativement, la classification de chacune des régions du cœur peut être réalisée selon l'une des modalités suivantes :
- elle est également fonction de la répétition au sein de l'enregistrement régional correspondant des lignes de base à ratio calculé de valeurs répétitives ; une région cible étant un vrai positif si la répétition est avérée ;
- elle est également fonction de la moyenne des valeurs d'amplitudes des dépolarisations de l'enregistrement régional correspondant ; une région cible étant un vrai positif si la moyenne des valeurs d'amplitudes est inférieure à une valeur seuil donnée ;
- elle est également fonction de la répétition au sein de l'enregistrement régional correspondant des lignes de base à ratio calculé de valeurs répétitives et fonction de la moyenne des valeurs d'amplitudes des dépolarisations de l'enregistrement régional correspondant ;une région cible étant un vrai positif si la répétition est avérée et si la moyenne des valeurs d'amplitudes est supérieure à une valeur seuil donnée.

Selon un autre aspect de l'invention, il est proposé un appareil pour l'identification de zones du cœur susceptibles d'être impliquées dans la perpétuation de la fibrillation atriale, comprenant un analyseur numérique et un cartographe numérique,
l'analyseur numérique étant adapté pour mettre en œuvre le procédé décrit ci-dessus ;
le cartographe numérique étant adapté pour générer une carte du cœur du patient sur laquelle la classification des zones du cœur est mise en forme.

Les étapes du procédé décrit ci-dessus peuvent être mises en œuvre par un programme d'ordinateur comprenant une suite d'instructions lorsque celles-ci sont exécutées par un processeur.

Ce programme d'ordinateur peut être enregistré sur un support de stockage non-transitoire et lisible par ordinateur.

### Présentation des dessins

D'autres objectifs, caractéristiques et avantages apparaitront à la lecture de la description détaillée qui suit en référence aux dessins données à titre illustratif et non limitatif, parmi lesquels :
- la figure 1 est un schéma illustrant la propagation de la dépolarisation des cellules d'un cœur ne souffrant pas d'arythmie cardiaque ;
- la figure 2 est un schéma illustrant la propagation de la dépolarisation des cellules d'un cœur souffrant d'arythmie cardiaque et présentant des rétro-boucles **R** ;
- la figure 3 représente de manière schématique les étapes du procédé selon l'invention ;
- la figure 4 montre un exemple d'électrogramme en zone saine (dépolarisations fines et amples non complexes) ;
- la figure 5 montre un exemple d'électrogramme complexe correspondant à un CFAE continue, c'est-à-dire n'ayant pas de ligne de base identifiable ;
- la figure 6 montre un exemple d'électrogramme fragmentés non continus répétitifs (appelés « train de CFAE ») avec une succession de lignes de base de durée inférieure au cycle de référence de l'arythmie de façon répétitive (non isolée) ;
- la figure 7 montre un exemple d'électrogrammes fragmentés avec lignes de bases courtes (donc durée d'événement de dépolarisation longue) mais de façon isolée (sans stabilité temporelle).
- la figure 8 montre un exemple d'électrogramme non fragmenté avec une ligne de base de durée inférieure au cycle de référence de l'arythmie (appelé « *rapid fire* »);
- la figure 9 montre un ensemble d'électrogrammes locaux correspondant à une région du cœur du patient à partir desquels un électrogramme régional peut être obtenu ; et
- la figure 10 illustre de manière schématique un appareil pour l'identification de zones du cœur susceptibles d'être impliquées dans une arythmie cardiaque selon l'invention.

### Description détaillée

### Analyse monopiste

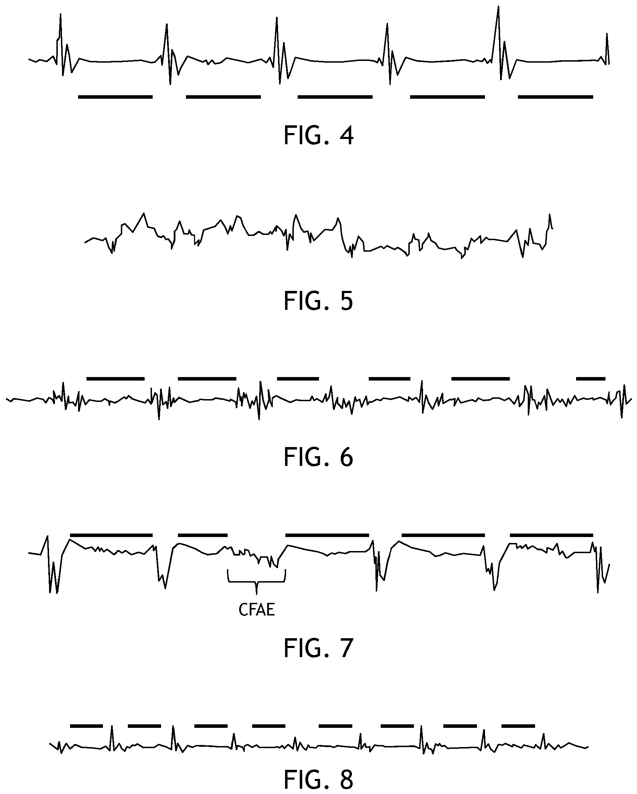

Dans le présent exposé, il sera question d'enregistrements de l'activité électrique (potentiel) d'une zone du cœur placée entre deux électrodes (ou dipôle) pendant une fenêtre de mesure. L'activité électrique du cœur se manifeste par une ou plusieurs dépolarisations par rapport à une ligne de base. Ainsi, on représente de manière générale un enregistrement, par une ligne continue dessinant des dépolarisations, c'est-à-dire des déflexions de la ligne de base, dont la base est plus ou moins large et l'amplitude plus ou moins haute (voir figure 4). Entre deux dépolarisations, une portion fluctuant seulement légèrement est généralement présente. Une telle portion est la ligne de base. On appellera « électrogrammes » ou EGM les dépolarisations par rapport à la ligne de base. Dans le cas où une durée inférieure à 35 ms, préférentiellement 30 ms sépare deux dépolarisations, ces deux dépolarisations sont alors considérées, pour l'analyse, comme une succession de dépolarisations qui appartient à un seul et même EGM.

L'enregistrement peut être la suite de mesures brutes, c'est-à-dire tel qu'ilest obtenu sans traitement pendant ou postérieurement à la mesure. L'enregistrement est de préférence la suite de mesures préalablement traitée, soit pendant, soit après la réalisation des mesures, afin notamment de supprimer le bruit dû en particulier aux équipements présents dans la salle, voire au voisinage de la salle où sont réalisés les enregistrements. Dans la pratique, l'activité électrique du cœur est mesurée à l'aide d'une paire d'électrodes (aussi appelée dipôle) recouvrant la zone correspondante. Pour faciliter la compréhension de l'ensemble du présent exposé, on distinguera les enregistrements locaux des enregistrements régionaux. Un enregistrement local est obtenu par mesure, notamment à l'aide d'un dipôle. Un enregistrement régional est obtenu par addition ou soustraction d'une pluralité d'enregistrements locaux correspondant à des zones adjacentes du cœur formant une région (*cf.* l'analyse multipiste décrite plus loin). Pareillement, on distinguera les EGM locaux des EGM régionaux. Un EGM local est un EGM d'un enregistrement local, alors qu'un EGM régional est un EGM d'un enregistrement régional.

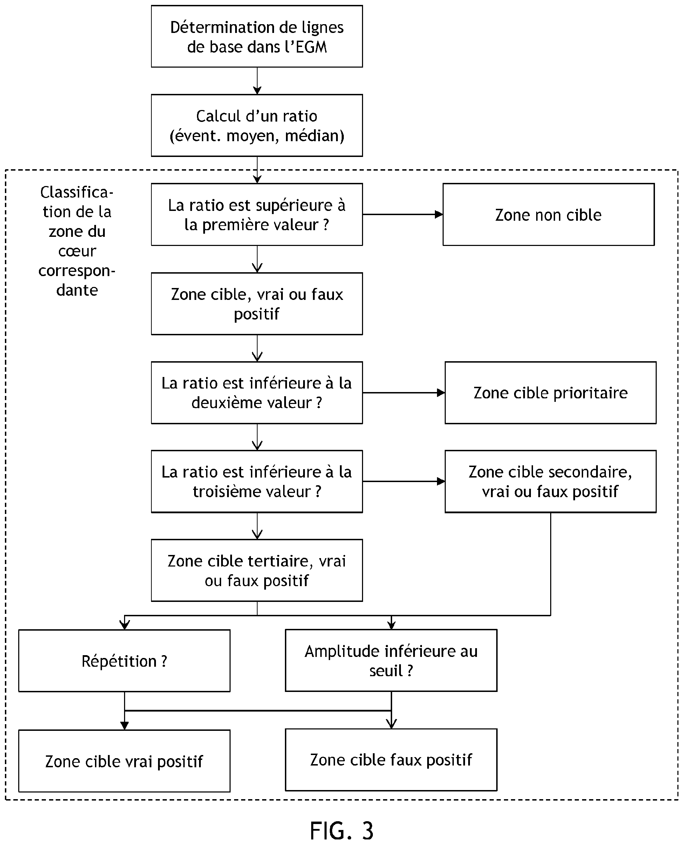

En référence à la figure 3, un procédé pour l'identification de zones du cœur susceptibles d'être impliquées dans la perpétuation de la FA (aussi appelée arythmie cardiaque) selon l'invention est décrit ci-après. Dans un mode de réalisation préférée, le procédé est effectué sur l'une des oreillettes du cœur ou les deux (droite et gauche).

Le procédé est réalisé à partir d'une pluralité d'enregistrements locaux réalisés sur le cœur du patient pendant une fenêtre de mesure, chaque enregistrement correspondant à une zone du cœur du patient. Chaque enregistrement local comprend au moins un EGM local. Il peut aussi par ailleurs présenter au moins une ligne de base.

La fenêtre de mesure est une fenêtre temporelle d'une durée déterminée et choisie suffisamment longue afin de pouvoir enregistrer plusieurs cycles de l'arythmie (par exemple plus de deux cycles de référence de l'arythmie).La fenêtre de mesure est avantageusement supérieure à 2 s, de préférence supérieure ou égale à 2,5 s. La fenêtre de mesure est soit temporellement continue, soit temporellement disjointe. Ainsi, la fenêtre de mesure peut également être deux périodes de temps qui ensemble ont une durée avantageusement supérieures à 2 s, de préférence supérieure ou égale à 2,5 s pendant lesquelles les mesures sont réalisées sur la même zone du cœur. Les mesures peuvent être répétées dans chaque zone pour améliorer la précision.

Bien entendu, la fenêtre de mesure est la même pour tous les enregistrements locaux ou régionaux considérés tant en terme de durée qu'en terme de l'instant de mesure.

De manière plus pratique, il est possible de considérer une portion comme ligne de base si sur toute la portion, la valeur du potentiel électrique mesurée est en dessous d'une valeur seuil inférieure, qui de manière générale est égale au moins au niveau de bruit maximal observé (notamment préalablement mesuré) pour l'équipement et son environnement, et est avantageusement égal au bruit maximal observé augmenté de 0,01 mV. La valeur seuil inférieure est de préférence comprise entre 0.02 mV et 0.04 mV, toujours de préférence d'environ 0.02 mV Les dépolarisations peuvent être isolées (généralement correspondant à une activité électrique normale d'un tissus « sain », en d'autres termes ce sont des potentiels non fragmentés) ou regroupées sans ligne de base intercalée entre eux (activités complexes avec plusieurs pics). Ainsi, une ligne de base commence à la fin d'une dépolarisation et se termine au début d'une autre dépolarisation subséquente.

Plus précisément, un enregistrement local mesuré dans une zone saine présente une succession de cycles constitués d'une activité basale représentée par une ligne de base fluctuant très légèrement, dont la durée est généralement supérieure à 120 ms, suivie d'un forte activité (EGM) conduisant à un changement d'amplitude fort et bref (dépolarisation) du potentiel mesurée (la durée est généralement inférieure à 40 ms) (figure 4). La durée des cycles est sensiblement constante.

En revanche, certains enregistrements peuvent présenter une forme s'éloignant de la forme d'un enregistrement local sain. Ces enregistrements, de forme anormale, présentent un fractionnement des dépolarisations, en effet, ils présentent au moins un EGM comprenant plus de trois dépolarisations qui se suivent rapidement (généralement dans un délai compris entre 2 et 35 ms) sans ligne de base intermédiaire identifiable, ces dépolarisations non isolées sont en outre d'amplitude plus faible. Ce type d'EGM sont appelés EGM atriaux fractionnés complexes (en anglais *complex fractionated atrial electrograms* aussi connus sous le sigle CFAE).

Cependant, bien que les CFAE soient le signe d'un événement anormal de l'activité électrique du cœur, ils ne préjugent en rien du caractère actif ou passif de la zone du cœur correspondante dans la perpétuation de la FA.

Deux types de CFAE dits actifs peuvent être distingués :
- le train de CFAE qui est une succession de CFAE ayant une durée supérieure ou égale à 50 ms (figure 6); et
- le CFAE continu qui est une succession de dépolarisation avec une quasi absence de ligne de base (figure 5).

D'autres enregistrements peuvent présenter des EGM ressemblant à des dépolarisations saines mais entre lesquels la durée de ligne de base est constamment courte, généralement inférieure à 120 ms (figure 8). L'amplitude des potentiels mesurés est généralement inférieure à celle des potentiels mesurés pour des dépolarisations saines, ils ne sont pas fragmentés et leur largeur est inférieure à 35 ms en général. Ces EGM sont dits à déclenchement rapide, ou plus communément *rapid fires* en anglais.

Les mesures peuvent être obtenues de différentes manières connues de l'homme du métier. Par exemple, elles sont obtenues en insérant une paire d'électrodes à l'intérieur du corps du patient, jusqu'à une zone du cœur intéressant le praticien, l'activité électrique de cette zone est enregistré pendant la fenêtre de temps choisie. La localisation de la zone est également enregistrée. À la fin de la fenêtre de temps, la paire d'électrodes est déplacée vers une autre zone du cœur intéressant le praticien, et l'enregistrement est effectué pour cette autre zone. Ceci est répété sur une pluralité de zones du cœur formant un maillage du cœur.

Alternativement, l'enregistrement peut être effectué à l'aide d'une sonde multi-électrodes comprenant une pluralité de paires d'électrodes permettant l'enregistrement de l'activité électrique d'une pluralité de zones du cœur en même temps. Ceci permet un enregistrement plus rapide et plus précis de toutes les zones du cœur ainsi que l'enregistrement simultané de plusieurs zones (permettant la création d'un EGM régional) ayant un intérêt pour le praticien. Par ailleurs, comme le temps de mesure est réduit, il est alors possible d'obtenir un maillage plus fin du cœur.

Les enregistrements peuvent être utilisés directement après mesure ou stockés en attendant être analysés par le présente procédé.

La particularité du présent procédé est de prendre en compte le cycle de référence de l'arythmie mesuré sur un ou plusieurs enregistrements locaux réalisés sur le patient afin d'analyser les enregistrements locaux de ce patient relativement à ce cycle. C'est pourquoi, le procédé comprend l'obtention d'un cycle de référence de l'arythmie du patient.

Le cycle de référence de l'arythmie est la durée moyenne des lignes de base entre deux EGM locaux d'un enregistrement local correspondant à une zone saine du cœur du patient. Avantageusement, le cycle de référence de l'arythmie est déterminé à partir des lignes de base prises à au moins deux moments différents dans un enregistrement local correspondant à une zone « saine » du cœur, c'est-à-dire une zone présentant un voltage d'amplitude d'au moins 0,5 mV avec des potentiels simples (dépolarisation normale de la membrane des cellules formant le tissu de la zone) dont la durée, de manière générale, n'excède pas 40 ms. Dans ces zones saines, les dépolarisations sont hautement instantanées (leur durée est généralement inférieure à 40 ms) de sorte que la durée de la ligne de base présente une grande correspondance avec le cycle total de l'activité électrique du tissus, d'où l'emploi du terme de *cycle.* Le cycle de référence de l'arythmie est mesuré sur l'oreillette droite, l'oreillette gauche ou le sinus coronaire du patient étant en fibrillation atriale (spontanée ou déclenchée intentionnellement si besoin). Le plus souvent ce cycle de référence est mesuré dans l'auricule gauche (petite poche dans l'oreillette gauche dont l'activité est en général saine).

Le cycle de référence de l'arythmie est de préférence la moyenne (par exemple d'au moins 10 cycles, voire 20 cycles) des durées de lignes de base dans un ou plusieurs enregistrements locaux sains.

De manière générale, le cycle de référence de l'arythmie diffère d'un patient à l'autre. Par ailleurs, sa durée est fonction de la complexité de l'arythmie, de l'état plus ou moins pathologique des oreillettes et de la présence de drogues anti-arythmiques. De manière générale, le cycle de référence de l'arythmie est compris entre environ 110 ms et environ 400 ms (en général entre environ 140 ms et environ-220 ms).

La fenêtre de mesure est avantageusement choisie de manière à permettre l'observation d'au moins deux, de préférence plus de deux, EGM dans l'enregistrement local correspondant à deux cycles de référence de l'arythmie (c'est-à-dire que leurs durées sont équivalentes d'un point de vue médical).

Le procédé comprend également, pour chaque enregistrement local, la détermination de lignes de base entre deux EGM locaux et le calcul d'au moins un ratio par rapport au cycle de référence de l'arythmie :
- pour chaque ligne de base, calcul du ratio de la durée de la ligne de base sur le cycle de référence de l'arythmie (ce qui signifie que si cette option est choisie, un ratio est calculé pour chaque ligne de base identifiée) ;
- un ratio moyen de la durée de la ligne de base sur le cycle de référence de l'arythmie ;
- un ratio médian de la durée de la ligne de base sur le cycle de référence de l'arythmie.

Ainsi, le procédé selon la présente invention repose sur le calcul d'un ratio dont le dénominateur est le cycle de référence de l'arythmie, aboutissant ainsi à une quantification automatique de ce ratio.

Le procédé comprend également la classification de chacune des zones selon leur degré d'implication dans la perpétuation de la FA. Le degré d'implication d'une zone est fonction du ou des ratios calculés pour l'enregistrement local correspondant.

Le procédé comprend enfin l'identification, à partir de la classification, des zones cibles.

Les zones peuvent être classées selon différentes échelles de degré d'implication. Ces échelles peuvent être plus ou moins fines.

Par exemple, une zone est classée comme une zone non-cible si le ratio moyen ou tous les ratios calculés pour les lignes de base de l'enregistrement local correspondant sont supérieurs à une première valeur, et en zone cible, vrai ou faux positif, sinon.

En d'autres termes, la zone non-cible correspondante a un degré d'implication faible et est une zone passive vis-à-vis de l'arythmie. Les zones cibles vrai ou faux positifs présentent généralement des enregistrements locaux avec des CFAE (continus ou « trains de CFAE ») ou des *rapidfires* si non fragmentés (figures 5,6 et 8 respectivement).

Autre exemple, une zone est classée comme une zone cible prioritaire vrai ou faux positif si le ratio moyen ou au moins un ratio calculé pour une ligne de base de l'EGM local correspondant est inférieur ou égale à une deuxième valeur, le cas échéant inférieure à la première valeur.

En d'autres termes, si aucune ligne de base n'est identifiable dans un enregistrement local (ratio calculé nul), ou si le ratio calculé est faible (par exemple inférieure à 35%), la zone correspondante a un degré d'implication très élevé dans la perpétuation de la FA (voir figure 5). En effet, une telle zone est probablement une zone de conduction lente (substrat des rotors), potentiellement le siège d'une rétro-boucle ou au moins une partie de celle-ci, ce qui correspond à une zone cible prioritaire. Une rétro-boucle ou encore réentrée caractérise un phénomène dans lequel la dépolarisation électrique, qui normalement se propage d'une zone du cœur à une autre de manière à permettre un battement de celui-ci (comme décrit plus haut dans l'introduction), se propage en boucle formant un circuit électrique. Ce circuit électrique s'auto-entretient perpétuant ainsi l'arythmie. Pour qu'un tel phénomène se produise, un ralentissement de conduction électrique (conduction lente) est nécessaire.

Dans le cas où les première et deuxième valeurs sont utilisées, les zones dont le ratio calculé est compris entre ces deux valeurs sont classées comme zones cibles vrais ou faux positifs.

En d'autres termes, la zone correspondante a un degré d'implication moyen à élevé.

Autre exemple, la classification peut être encore plus affinée. Pour cela, une zone est classée comme une zone cible secondaire vrai ou faux positif si :
- le ratio moyen, le ratio médian, ou aucun des ratios calculés pour les lignes de base de l'enregistrement local correspondant n'est inférieur à la deuxième valeur ; et
- le ratio moyen, le ratio médian, ou au moins un ratio calculé pour une ligne de base de l'enregistrement local correspondant est compris entre la deuxième valeur et une troisième valeur comprise entre les première et deuxième valeurs.

La zone est alors classée comme une zone cible tertiaire vrai ou faux positif si :
- le ratio moyen, le ratio médian, ou aucun des ratios calculés pour les lignes de base de l'enregistrement local correspondant n'est inférieur à la deuxième valeur ;
- le ratio moyen, le ratio médian, ou au moins un ratio calculé pour une ligne de base de l'enregistrement local correspondant est compris entre la troisième valeur et la première valeur.

Les valeurs numéraires des première, deuxième et troisième valeurs sont choisies en fonction des besoins, du patient, des circonstances de mesures, etc. Avantageusement, les valeurs pour les exemples ci-dessus sont :

| Première valeur | Deuxième valeur | Troisième valeur |
|---|---|---|
| 65% | 35% | 50% |

Néanmoins, ces valeurs ne sont qu'indicatives et le praticien pourra décider d'autres valeurs selon le cas.

Ainsi, préférentiellement, la classification comprend au moins quatre niveaux de base : cibles prioritaires, cibles secondaires, cibles tertiaires et non cibles (degrés d'implication du plus au moins élevés). Chacune de ces niveaux peuvent être sous divisés. Les sous-divisions des niveaux de base ne sont pas forcément identiques en nombre.

Les zones cibles prioritaires (de degré d'implication très élevé) sont des zones qu'il faudrait ablater en priorité car suspectées d'être des zones responsables de la perpétuation de la FA.

Ainsi la probabilité pour qu'elles soient responsables de la perpétuation de la FA est extrêmement forte.

Les zones cibles secondaires et tertiaires (de degré d'implication élevé à moyen) sont des zones éventuellement à ablater, le praticien choisit ou non de le faire, notamment en fonction du résultat de l'ablation des cibles prioritaire, mais leur classification indique une forte à moyenne suspicion de responsabilité dans la perpétuation et/ou initiation des arythmies cardiaques. Ainsi la probabilité pour qu'elles soient responsables de la perpétuation de la FA est forte à moyenne.

Les zones non cibles sont des zones de degré d'implication faible sont des zones qui ne devraient pas être ablatées car la probabilité pour qu'elles soient responsables de la perpétuation de la FA est extrêmement faible.

La fiabilité du procédé peut être améliorée par des critères supplémentaires avec les étapes suivantes. Ces étapes permettent de différencier les vrais des faux positifs parmi les zones cibles.

Dans une première variante, la classification de chacune des zones du cœur est également fonction de la répétition au sein de l'enregistrement local correspondant des lignes de base à ratio calculé de valeurs répétitives ; une zone cible étant un vrai positif si la répétition est avérée.

Le terme répétitif signifie que la durée des lignes de base sont égales à plus ou moins 15%, préférentiellement 10%, encore préférentiellement 5% les unes des autres.

En d'autres termes, il y a une stabilité temporelle dans la survenu des événements anormaux (EGM locaux anormaux) au sein de l'enregistrement local correspondant. C'est cette stabilité temporelle qui permet de préciser la classification. Cette stabilité temporelle est préférentiellement maintenue pour au moins 3 lignes de base successives, préférentiellement au moins 4 voire au moins 5 lignes de base successives).Les zones cibles faux positif passent alors en zones non-cibles.

Dans une autre variante, la classification de chacune des zones du cœur est également fonction de la moyenne des valeurs d'amplitudes (ou « voltage ») des dépolarisations de l'enregistrement local correspondant ;
une zone cible étant un vrai positif si la moyenne des valeurs d'amplitudes est inférieure à une valeur seuil donnée (généralement de 0,6 mV) et faux positif sinon. Les zones cibles faux positif passent alors en zones non-cibles.

Les deux variantes ci-dessus peuvent être avantageusement combinées. Dans ce cas, la classification de chacune des zones du cœur est également fonction de la répétition au sein de l'enregistrement local correspondant des lignes de base à ratio calculé de valeurs répétitives et de la moyenne des valeurs d'amplitudes des dépolarisations de l'EGM local correspondant ;
une zone cible étant un vrai positif si la répétition est avérée et si la moyenne des valeurs d'amplitudes est inférieure à une valeur seuil donnée, elle est un faux positif sinon. Les zones cibles faux positif passent alors en zones non-cibles.

Par ailleurs, le procédé peut encore prendre en compte la durée des EGM locaux afin notamment de distinguer les trains de CFAE et les *rapid fires.* Pour cela, la classification est également fonction de la durée des durées des EGM locaux, notamment ceux des enregistrements correspondant à des zones cibles vrais ou faux positifs, qu'elles soient prioritaires, secondaires ou tertiaires. Si la durée de l'EGM est inférieure à une durée seuil d'EGM local donnée, l'EGM est classé comme *rapid fire* ; sinon, l'EGM local est classé comme train de CFAE. La durée seuil d'EGM local donnée est généralement choisie inférieure à 45 ms, préférentiellement à 35 ms, toujours préférentiellement à 30 ms. La distinction des trains de CFAE et des *rapid fires* peut être utile, notamment à des fins de recherche.Le procédé comprend également la mise en forme de la classification sur une carte du cœur du patient. La mise en forme comprend toutes les méthodes possibles de représentation des degrés d'implication des zones sur une carte des oreillettes du patient, par exemple deux degrés d'implication peuvent différer par deux couleurs différentes, deux tons différent d'une même couleur, deux motifs différents, voire par des moyens dynamiques tels que la présence ou non d'un scintillement, la fréquence du scintillement, etc.

Le procédé peut en outre comprendre une étape d'affichage de la carte des oreillettes ainsi obtenue. La carte des oreillettes affichée peut montrer toutes les zones des oreillettes pour lesquelles l'activité électrique a été mesurée ou seulement une partie d'entre elles. Par exemple, il est possible de choisir d'afficher seulement les zones de degré d'implication très élevé, ou les zones de degré d'implication élevé, ou les zones de degré d'implication faible, ou un mélange de ceux-ci.

Ci-dessus, le procédé correspond à une analyse monopiste, c'est-à-dire que les enregistrements locaux sont classés individuellement.

### Analyse multipiste

Bien qu'une analyse monopiste donne des résultats satisfaisants, ceux-ci peuvent encore être améliorés grâce à une analyse multipiste décrite ci-après, notamment en ce qui concerne les zones classées comme non-cible par l'analyse monopiste.

Une analyse multipiste consiste avant tout à identifier des régions du cœur où se produisent des rotors. En effet, il se peut que lors de l'analyse mono-piste sur des zones d'une région, aucune zone n'est classée comme cible que ce soit vrai ou faux positif car le ratio calculé est supérieur à la première valeur, alors qu'en réalité, du fait de la présence d'un rotor, l'ensemble des événements témoignant de la FA est réparti sur une région regroupant plusieurs zones adjacentes.

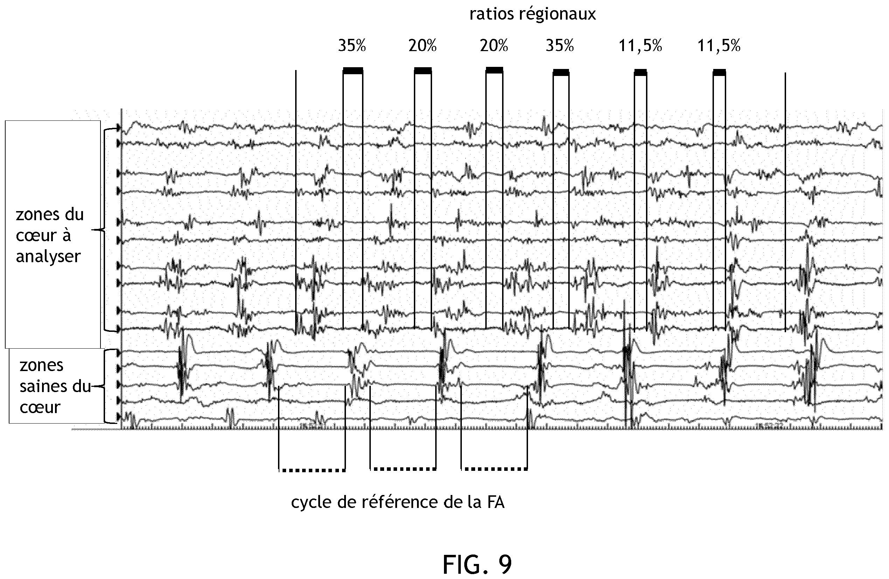

Afin de prendre en compte cette possibilité, le procédé peut comprendre la génération d'au moins un enregistrement régional à partir d'une pluralité d'enregistrements locaux correspondant à des zones adjacentes (voir figure 9), notamment par addition de la pluralité d'enregistrements locaux (ou soustraction des lignes de bases correspondantes). Les zones adjacentes forment alors une région correspondant à l'enregistrement régional.

Par le terme « addition(ner) » on entend ici le fait d'additionner les mesures correspondant aux enregistrements locaux en question ou le fait de superposer les enregistrements locaux en question les uns sur les autres.

Ainsi, comme on peut le voir sur la figure 9, lorsque les enregistrements locaux sont additionnés pour générer un enregistrement régional, on obtient des EGM régionaux formés à partir d'au moins un EGM local, (dans le cas d'une pluralité d'EGM locaux, chaque EGM local se trouve généralement sur un enregistrement local distinct d'un autre). Ainsi, ces EGM régionaux présentent une durée supérieure ou égale à la durée des EGM locaux.

Les lignes de base obtenues pour cet enregistrement régional sont analysées relativement au cycle de référence de l'arythmie. Cependant, il faut préciser que les enregistrements locaux ne présentant aucune ligne de base identifiable doivent être écartés (les zones correspondantes sont des zones cibles prioritaires suite à l'analyse monopiste). La suite de l'exposé prend en compte cette dernière remarque.

Le procédé est ensuite réalisé en substituant les enregistrements locaux par les enregistrements régionaux. En d'autres termes :
- pour chaque enregistrement régional, le procédé comprend :
   ▪ la détermination de lignes de base entre deux dépolarisations dans l'enregistrement régional ;
   ▪ le calcul d'au moins un ratio parmi :
      ∘ un ratio de la durée de la ligne de base sur le cycle de référence de l'arythmie pour chaque ligne de base;
      ∘ un ratio moyen ou médian de la durée de la ligne de base sur le cycle de référence de l'arythmie ; et
   ▪ la classification de la région du cœur correspondante selon son degré d'implication dans la perpétuation de la FA, le degré d'implication de la région étant fonction du ou des ratios calculés.

Ceci comprend également le cas où aucun enregistrement régional n'est réellement obtenu par addition des enregistrements locaux (ou soustraction des lignes de bases) des zones correspondantes, mais seulement en déterminant dans les enregistrements locaux une portion où tous les enregistrements locaux des zones de la région présentent une partie ou la totalité d'une ligne de base.

Les autres étapes décrites ci-dessus sont également applicables ici pour les enregistrements régionaux. Pour cela, il suffit de substituer les enregistrements locaux par les enregistrements régionaux et les zones du cœur par les régions du cœur.

Par ailleurs, le procédé peut être réalisé en faisant l'économie des calculs sur les enregistrements locaux. En d'autres termes, les étapes du procédé ne sont réalisées qu'à partir des enregistrements régionaux.

Un tel procédé est donc un procédé pour l'identification de régions du cœur d'un patient susceptibles d'être impliquées dans la perpétuation de la fibrillation atriale, ci-après régions cibles. Le procédé part d'une pluralité d'enregistrements régionaux réalisés sur le cœur du patient pendant une fenêtre de mesure, chaque enregistrement régional correspondant à une région du cœur du patient, le procédé comprenant les étapes suivantes :
- l'obtention d'un cycle de référence de l'arythmie, le cycle de référence étant la durée moyenne des lignes de base entre deux dépolarisations d'un enregistrement local correspondant à une zonesaine du cœur du patient ; et
- pour chaque enregistrement régional :
   ▪ la détermination de lignes de base entre deux dépolarisations dans l'enregistrement régional ;
   ▪ le calcul d'au moins un ratio parmi :
      ∘ un ratio moyen de la durée de la ligne de base sur le cycle de référence de l'arythmie ;
      ∘ pour chaque ligne de base, un ratio de la durée de la ligne de base sur le cycle de référence de l'arythmie ;
   ▪ la classification de la région du cœur correspondante selon son degré d'implication dans la perpétuation de la FA, le degré d'implication de la région étant fonction du ou des ratios calculés.

Là encore, l'enregistrement régional est obtenu par addition d'enregistrements locaux (ou soustraction de lignes de bases correspondantes) des zones du cœur dans la région donnée, sachant que les enregistrements locaux ne présentant aucune ligne de base identifiable doivent être écartés (car ils correspondent à des zones cibles prioritaires, voir explication dans la partie analyse monopiste).

Là aussi, les autres étapes décrites ci-dessus sont également applicables ici pour les enregistrements régionaux. Pour cela, il suffit de substituer les enregistrements locaux par les enregistrements régionaux et les zones du cœur par les régions du cœur.

Avantageusement, le regroupement des zones par régions est réalisé de manière à ce qu'une région comprend une pluralité de zones adjacentes, généralementau moins 5 zones, de préférence au moins 10 zones, encore de préférence entre 20 et 50 zones.

Dans un tel cas, deux zones sont séparées d'une distance d'au moins 4 mm, de préférence inférieure à 2 mm. La distance étant prise entre les centres des zones.

De préférence, une analyse multipiste est réalisée quand les mesures ont été enregistrées avec une sonde multi-électrodes. Dans ce cas, il est possible et aisé de considérer que l'étendu couvert par les électrodes de la sonde multi-électrodes constitue une régiondont la surface est préférentiellement de l'ordre de 7 cm².

L'enregistrement régional peut être généré sur toute la fenêtre de mesure mais également sur une portion de celle-ci. En d'autre terme c'est la première dépolarisation précédée d'une ligne de base subséquente à la dépolarisation suivie d'une ligne de base sur le premier enregistrement local qui soit identifiable parmi tous les enregistrements locaux des zones de la région.

Le procédé prend également en compte le délai entre deux EGM différents, en effet, celui-ci est fonction de la distance entre les deux dipôles (chaque dipôle permettant la mesure d'un EGM d'une zone). Le calcul du ratio sera alors adapté à ce délai. Par ailleurs, il est préférable, mais non nécessaire, que la distance entre deux zones d'une même région n'excède pas environ 2 cm voire 3 cm.

Si une sonde de plus grande surface était utilisée une formule devra adapter le ratio limite de cible prioritaire à la distance.

En pratique, étant donné que ces réentrées sont instables et donc mobiles localement dans une région. Si la surface de la sonde n'excède pas 7 cm² il n'est pas nécessaire de pondérer le calcul du ratio de l'EGM régional en fonction de la distance entre les dipôles d'électrodes.

### Appareil

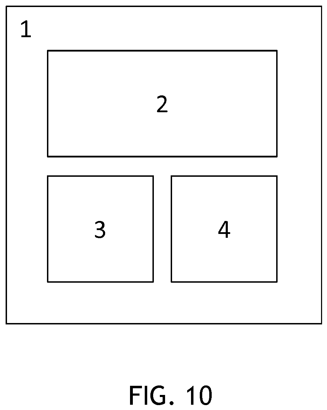

En référence à la figure 10, un appareil **1** pour l'identification de zones du cœur susceptibles d'être impliquées dans une arythmie cardiaque est décrit ci-après. Cet appareil **1** est adapté pour mettre en œuvre le procédé décrit ci-dessus.

Cet appareil **1** comprend un analyseur numérique **2** et un cartographe numérique **3.**

L'analyseur numérique **2** est adapté pour recevoir :
- d'un cycle de référence de l'arythmie, le cycle de référence étant la durée moyenne des lignes de base entre deux dépolarisations d'un EGM local correspondant à une zone saine du cœur du patient ; et
- une pluralité d'EGM locaux ou réalisés sur le cœur du patient pendant une fenêtre de mesure, chaque EGM locaux correspondant à une zone du cœur du patient.

Pour cela, l'analyseur numérique **2** peut comprendre un module de réception.

L'analyseur numérique **2** peut être adapté pour générer des EGM régionaux à partir d'une pluralité d'EGM locaux correspondant à des zones adjacentes du cœur. Pour cela, l'analyseur numérique **2** peut comprendre un générateur d'EGM régionaux.

L'analyseur numérique **2** est également adapté pour déterminer des lignes de base entre deux dépolarisations dans un EGM local et éventuellement régional.

L'analyseur numérique **2** est également adapté, pour un EGM local ou régional, pour le calcul d'au moins un ratio parmi :
▪ un ratio moyen de la durée de la ligne de base sur le cycle de référence de l'arythmie ;
▪ pour chaque ligne de base, un ratio de la durée de la ligne de base sur le cycle de référence de l'arythmie.

Pour cela, l'analyseur numérique **2** peut comprendre un calculateur.

L'analyseur numérique**2** est également adapté, pour un EGM local ou régional, pour la classification de la zone ou région du cœur correspondante selon son degré d'implication dans la perpétuation de la FA, le degré d'implication de la zone ou région étant fonction du ou des ratios calculés. Les détails de la classification sont décrits ci-dessus dans la partie descriptive du procédé. Pour réaliser la classification, l'analyseur numérique **2** peut comprendre un classificateur.

Le cartographe numérique **3** est adapté pour générer une carte du cœur du patient sur laquelle la classification des zones ou régions du cœur est mise en forme.

L'appareil comprend en outre un afficheur **4** pour afficher la carte du cœur du patient.

### Programme d'ordinateur

Le procédé décrit plus haut peut être mis en œuvre par un programme d'ordinateur. Ce programme d'ordinateur comprend une suite d'instructions qui mettent en œuvre les étapes du procédé décrit ci-dessus lorsqu'elles sont exécutées par un processeur.

Ce programme d'ordinateur peut être stocké sur un support de stockage non-transitoire et lisible par ordinateur. Ce type de support de stockage peut être par exemple un CD, un DVD, une clef USB, une SimCard etc.

## Revendications

1. Procédé pour l'identification de zones du cœur d'un patient susceptibles d'être impliquées dans la perpétuation de la fibrillation atriale mis en œuvre par un processeur, ci-après zones cibles, à partir d'une pluralité d'enregistrements locaux réalisés sur le cœur du patient pendant une fenêtre de mesure, chaque enregistrement local correspondant à une zone du cœur du patient, chaque enregistrement local comprenant au moins un EGM local et éventuellement au moins une ligne de base, le procédé comprenant les étapes suivantes :
- la réception d'un cycle de référence de l'arythmie, le cycle de référence étant la durée moyenne des lignes de base entre deux EGM locaux d'un enregistrement local correspondant à une zone saine du cœur du patient ; et
- pour chaque enregistrement local :
▪ la détermination de lignes de base entre deux EGM locaux ;
▪ le calcul d'au moins un ratio parmi :
∘ un ratio moyen de la durée de la ligne de base sur le cycle de référence de l'arythmie ;
∘ un ratio médian de la durée de la ligne de base sur le cycle de référence de l'arythmie ;
∘ pour chaque ligne de base, un ratio de la durée de la ligne de base sur le cycle de référence de l'arythmie ;
▪ la classification de la zone du cœur correspondante selon son degré d'implication dans la perpétuation de la FA, le degré d'implication de la zone étant fonction du ou des ratios calculés.

2. Procédé selon la revendication 1, dans lequel une zone est classée comme une zone non-cible si le ratio moyen, le ratio médian, ou tous les ratios calculés pour les lignes de base de l'enregistrement local correspondant sont supérieurs ou égaux à une première valeur,
la zone étant classée comme une zone cible vrai ou faux positif sinon.

3. Procédé selon la revendication 1 ou la revendication 2, dans lequel une zone est classée comme une zone cible prioritaire vrai ou faux positif si le ratio moyen, le ratio médian, ou au moins un ratio calculé pour une ligne de base de l'enregistrement local correspondant est inférieur ou égal à une deuxième valeur, le cas échéant inférieure à la première valeur.

4. Procédé selon la combinaison des revendications 2 et 3, dans lequel une zone est classée comme une zone cible secondaire vrai ou faux positif si le ratio moyen, le ratio médian, ou au moins un ratio calculé pour une ligne de base de l'enregistrement local correspondant est compris entre la deuxième valeur et une troisième valeur comprise entre les première et deuxième valeurs ; et
la zone est classée comme une zone cible tertiaire vrai ou faux positif si le ratio moyen, le ratio médian, ou au moins un ratio calculé pour une ligne de base de l'enregistrement local correspondant est compris entre la troisième valeur et la première valeur.

5. Procédé selon l'une des revendications 1 à 4, dans lequel la classification de chacune des zones du cœur est également fonction de la répétition au sein de l'enregistrement local correspondant des lignes de base à ratio calculé de valeurs répétitives ;
une zone cible étant un vrai positif si la répétition correspondant à la valeur de ratio ayant conduit à la classification de la zone comme étant une zone cible est avérée.

6. Procédé selon l'une des revendications 1 à 4, dans lequel la classification de chacune des zones du cœur est également fonction de la moyenne des valeurs d'amplitudes des dépolarisations de l'enregistrement local correspondant ;
une zone cible étant un vrai positif si la moyenne des valeurs d'amplitudes est inférieure à une valeur seuil donnée.

7. Procédé selon l'une des revendications 1 à 4, dans lequel la classification de chacune des zones du cœur est également fonction de la répétition au sein de l'enregistrement local correspondant des lignes de base à ratio calculé de valeurs répétitives et fonction de la moyenne des valeurs d'amplitudes des dépolarisations de l'enregistrement local correspondant ;
une zone cible étant un vrai positif si la répétition est avérée et si la moyenne des valeurs d'amplitudes est supérieure à une valeur seuil donnée.

8. Procédé selon l'une des revendications 1 à 7, comprenant en outre pour au moins une partie des enregistrements locaux du cœur :
- l'obtention d'au moins un enregistrement régional à partir d'une pluralité d'enregistrement locaux correspondant à des zones adjacentes, les zones adjacentes formant une région correspondant à l'enregistrement régional, les enregistrements locaux ne présentant aucune ligne de base identifiable étant écartés, ainsi l'enregistrement régional comprend au moins un EGM régional ; et
- pour chaque enregistrement régional :
▪ la détermination de lignes de base entre deux EGM régionaux ;
▪ le calcul d'au moins un ratio parmi :
∘ un ratio moyen de la durée de la ligne de base sur le cycle de référence de l'arythmie ; et
∘ pour chaque ligne de base, un ratio de la durée de la ligne de base sur le cycle de référence de l'arythmie ;
▪ la classification de la région du cœur correspondante selon son degré d'implication dans la perpétuation de la FA, le degré d'implication de la région étant fonction du ou des ratios calculés ;
les autres étapes du procédés étant réalisées en remplaçant :
- enregistrement local par enregistrement régional et
- zone par la région.

9. Procédé pour l'identification de régions du cœur d'un patient susceptibles d'être impliquées dans la perpétuation de la fibrillation atriale, ci-après régions cibles mis en œuvre par un processeur, à partir d'une pluralité d'enregistrements régionaux réalisés sur le cœur du patient pendant une fenêtre de mesure, chaque enregistrement régional correspondant à une région du cœur du patient regroupant une pluralité de zones, le procédé comprenant les étapes suivantes :
- la réception d'un cycle de référence de l'arythmie, le cycle de référence étant la durée moyenne des lignes de base entre deux dépolarisations d'un enregistrement local correspondant à une zone saine du cœur du patient ; et
- pour chaque enregistrement régional :
▪ la détermination de lignes de base entre les dépolarisations des enregistrements régionaux ;
▪ le calcul d'au moins un ratio parmi :
∘ un ratio moyen de la durée de la ligne de base sur le cycle de référence de l'arythmie ;
∘ pour chaque ligne de base, un ratio de la durée de la ligne de base sur le cycle de référence de l'arythmie ;
▪ la classification de la région du cœur correspondante selon son degré d'implication dans la perpétuation de la FA, le degré d'implication de la région étant fonction du ou des ratios calculés.

10. Procédé selon la revendication 9, dans lequel une région est classée comme une région non-cible si le ratio moyen ou si tous les ratios calculés pour les lignes de base de l'enregistrement régional correspondant est/sont supérieur(s) ou égal/égaux à une première valeur,
la région étant classée comme une région cible vrai ou faux positif sinon.

11. Procédé selon la revendication 9 ou la revendication 10, dans lequel une région est classée comme une région cible prioritaire vrai ou faux positif si le ratio moyen ou au moins un ratio calculé pour une ligne de base de l'enregistrement régional correspondant est inférieur ou égal à une deuxième valeur, le cas échéant inférieure à la première valeur.

12. Procédé selon la combinaison des revendications 10 et 11, dans lequel une région est classée comme une région cible secondaire vrai ou faux positif si le ratio moyen ou au moins un ratio calculé pour une ligne de base de l'enregistrement régional correspondant est compris entre la deuxième valeur et une troisième valeur comprise entre les première et deuxième valeurs ; et
la région est classée comme une région cible tertiaire vrai ou faux positif si le ratio moyen ou au moins un ratio calculé pour une ligne de base de l'enregistrement régional correspondant est compris entre la troisième valeur et la première valeur.

13. Procédé selon l'une des revendications 9 à 12, dans lequel la classification de chacune des régions du cœur est également fonction de la répétition au sein de l'enregistrement régional correspondant des lignes de base à ratio calculé de valeurs répétitives ;
une région cible étant un vrai positif si la répétition est avérée.

14. Procédé selon l'une des revendications 9 à 12, dans lequel la classification de chacune des régions du cœur est également fonction de la moyenne des valeurs d'amplitudes des dépolarisations de l'enregistrement régional correspondant ;
une région cible étant un vrai positif si la moyenne des valeurs d'amplitudes est inférieure à une valeur seuil donnée.

15. Procédé selon l'une des revendications 9 à 12, dans lequel la classification de chacune des régions du cœur est également fonction de la répétition au sein de l'enregistrement régional correspondant des lignes de base à ratio calculé de valeurs répétitives et fonction de la moyenne des valeurs d'amplitudes des dépolarisations de l'enregistrement régional correspondant ;
une région cible étant un vrai positif si la répétition est avérée et si la moyenne des valeurs d'amplitudes est supérieure à une valeur seuil donnée.

16. Appareil pour l'identification de zones du cœur susceptibles d'être impliquées dans la perpétuation de la fibrillation atriale, comprenant un analyseur numérique et un cartographe numérique, l'analyseur numérique étant adapté pour mettre en œuvre le procédé selon l'une des revendications 1 à 15 ;
le cartographe numérique étant adapté pour générer une carte du cœur du patient sur laquelle la classification des zones du cœur est mise en forme.

17. Programme d'ordinateur comprenant une suite d'instructions qui, lorsque elles sont exécutées par un processeur, conduisent celui-ci à mettre en œuvre les étapes du procédé selon l'une des revendications 1 à 15.

18. Support de stockage non-transitoire et lisible par ordinateur comprenant un programme d'ordinateur selon la revendication 17.

## Patentansprüche

1. Verfahren zum Identifizieren von Bereichen des Herzens eines Patienten, die an der Aufrechterhaltung des Vorhofflimmerns beteiligt sein könnten, nachstehend Zielbereiche genannt, das von einem Prozessor auf Grundlage einer Vielzahl von lokalen Aufzeichnungen umgesetzt wird, die während eines Messfensters am Herzen des Patienten vorgenommen werden, wobei jede lokale Aufzeichnung einem Bereich des Herzens des Patienten entspricht, wobei jede lokale Aufzeichnung mindestens ein lokales EGM und gegebenenfalls mindestens eine Basislinie umfasst, wobei das Verfahren die folgenden Schritte umfasst:
- das Empfangen eines Referenzzyklus der Arrhythmie, wobei es sich bei dem Referenzzyklus um die durchschnittliche Dauer der Basislinien zwischen zwei lokalen EGM einer lokalen Aufzeichnung handelt, die einem gesunden Bereich des Herzens des Patienten entspricht; und
- für jede lokale Aufzeichnung:
▪ das Bestimmen von Basislinien zwischen zwei lokalen EGM;
▪ das Berechnen von mindestens einem Verhältnis aus:
∘ einem Durchschnittsverhältnis der Dauer der Basislinie zum Referenzzyklus der Arrhythmie;
∘ einem Medianverhältnis der Dauer der Basislinie zum Referenzzyklus der Arrhythmie;
∘ für jede Basislinie, einem Verhältnis der Dauer der Basislinie zum Referenzzyklus der Arrhythmie;
▪ das Klassifizieren des entsprechenden Bereichs des Herzens gemäß dessen Beteiligungsgrad an der Aufrechterhaltung des VHF, wobei der Beteiligungsgrad des Bereichs von dem oder den berechneten Verhältnissen abhängig ist.

2. Verfahren nach Anspruch 1, wobei ein Bereich als ein Nicht-Zielbereich klassifiziert wird, wenn das Durchschnittsverhältnis, das Medianverhältnis, oder alle die Verhältnisse, die für die Basislinien der entsprechenden lokalen Aufzeichnung berechnet wurden, größer oder gleich einem ersten Wert sind,
wobei der Bereich andernfalls als ein richtig oder falsch positiver Zielbereich klassifiziert wird.

3. Verfahren nach Anspruch 1 oder Anspruch 2, wobei ein Bereich als ein richtig oder falsch positiver prioritärer Zielbereich klassifiziert wird, wenn das Durchschnittsverhältnis, das Medianverhältnis, oder mindestens ein Verhältnis, das für eine Basislinie der entsprechenden lokalen Aufzeichnung berechnet wurde, kleiner oder gleich einem zweiten Wert, gegebenenfalls kleiner als der erste Wert ist.

4. Verfahren nach der Kombination der Ansprüche 2 und 3, wobei ein Bereich als ein richtig oder falsch positiver sekundärer Zielbereich klassifiziert wird, wenn das Durchschnittsverhältnis, das Medienverhältnis, oder mindestens ein Verhältnis, das für eine Basislinie der entsprechenden lokalen Aufzeichnung berechnet wurde, zwischen dem zweiten Wert und einem dritten Wert liegt, der zwischen dem ersten und zweiten Wert liegt; und
der Bereich als ein richtig oder falsch positiver tertiärer Zielbereich klassifiziert wird, wenn das Durchschnittsverhältnis, das Medianverhältnis, oder mindestens ein Verhältnis, das für eine Basislinie der entsprechenden lokalen Aufzeichnung berechnet wurde, zwischen dem dritten Wert und dem ersten Wert liegt.

5. Verfahren nach einem der Ansprüche 1 bis 4, wobei die Klassifizierung jedes der Bereiche des Herzens ebenfalls von der Wiederholung der Basislinien mit einem berechneten Verhältnis sich wiederholender Werte innerhalb der entsprechenden lokalen Aufzeichnung abhängig ist;
wobei es sich bei einem Zielbereich um einen richtig Positiven handelt, wenn die Wiederholung, die dem Verhältniswert entspricht, der zur Klassifizierung des Bereichs als ein Zielbereich geführt hat, nachgewiesen wird.

6. Verfahren nach einem der Ansprüche 1 bis 4, wobei die Klassifizierung jedes der Bereiche des Herzens ebenfalls vom Durchschnitt der Amplitudenwerte der Depolarisationen der entsprechenden lokalen Aufzeichnung abhängig ist;
wobei es sich bei einem Zielbereich um einen richtig Positiven handelt, wenn der Durchschnitt der Amplitudenwerte kleiner als ein gegebener Schwellenwert ist.

7. Verfahren nach einem der Ansprüche 1 bis 4, wobei die Klassifizierung jedes der Bereiche des Herzens ebenfalls von der Wiederholung der Basislinien mit einem berechneten Verhältnis sich wiederholender Werte innerhalb der entsprechenden lokalen Aufzeichnung abhängig ist, und vom Durchschnitt der Amplitudenwerte der Depolarisationen der entsprechenden lokalen Aufzeichnung abhängig ist;
wobei es sich bei einem Zielbereich um einen richtig Positiven handelt, wenn die Wiederholung nachgewiesen wird, und wenn der Durchschnitt der Amplitudenwerte größer als ein gegebener Schwellenwert ist.

8. Verfahren nach einem der Ansprüche 1 bis 7, das für mindestens einen Teil der lokalen Aufzeichnungen des Herzens weiter umfasst:
- das Erhalten mindestens einer regionalen Aufzeichnung auf Grundlage einer Vielzahl von lokalen Aufzeichnungen, die angrenzenden Bereichen entsprechen, wobei die angrenzenden Bereiche eine Region bilden, die der regionalen Aufzeichnung entspricht, wobei die lokalen Aufzeichnungen, die keine identifizierbare Basislinie aufweisen, verworfen werden, sodass die regionale Aufzeichnung mindestens ein regionales EGM umfasst; und
- für jede regionale Aufzeichnung:
▪ das Bestimmen von Basislinien zwischen zwei regionalen EGM;
▪ das Berechnen von mindestens einem Verhältnis aus:
∘ einem Durchschnittsverhältnis der Dauer der Basislinie zum Referenzzyklus der Arrhythmie; und
∘ für jede Basislinie, einem Verhältnis der Dauer der Basislinie zum Referenzzyklus der Arrhythmie;
▪ das Klassifizieren der entsprechenden Region des Herzens gemäß deren Beteiligungsgrad an der Aufrechterhaltung des VHF, wobei der Beteiligungsgrad der Region von dem oder den berechneten Verhältnissen abhängig ist;
wobei die anderen Schritte des Verfahrens ausgeführt werden unter Ersetzen von:
- lokaler Aufzeichnung durch regionale Aufzeichnung, und
- Bereich durch die Region.

9. Verfahren zum Identifizieren von Regionen des Herzens eines Patienten, die an der Aufrechterhaltung des Vorhofflimmerns beteiligt sein könnten, nachstehend Zielregionen genannt, das von einem Prozessor auf Grundlage einer Vielzahl von regionalen Aufzeichnungen umgesetzt wird, die während eines Messfensters am Herzen des Patienten vorgenommen werden, wobei jede regionale Aufzeichnung einer Region des Herzens des Patienten entspricht, die eine Vielzahl von Bereichen zusammenfasst, wobei das Verfahren die folgenden Schritte umfasst:
- das Empfangen eines Referenzzyklus der Arrhythmie, wobei es sich bei dem Referenzzyklus um die durchschnittliche Dauer der Basislinien zwischen zwei Depolarisationen einer lokalen Aufzeichnung handelt, die einem gesunden Bereich des Herzens des Patienten entspricht; und
- für jede regionale Aufzeichnung:
▪ das Bestimmen von Basislinien zwischen den Depolarisationen der regionalen Aufzeichnungen;
▪ das Berechnen von mindestens einem Verhältnis aus:
∘ einem Durchschnittsverhältnis der Dauer der Basislinie zum Referenzzyklus der Arrhythmie;
∘ für jede Basislinie, einem Verhältnis der Dauer der Basislinie zum Referenzzyklus der Arrhythmie;
▪ das Klassifizieren der entsprechenden Region des Herzens gemäß deren Beteiligungsgrad an der Aufrechterhaltung des VHF, wobei der Beteiligungsgrad der Region von dem oder den berechneten Verhältnissen abhängig ist.

10. Verfahren nach Anspruch 9, wobei eine Region als eine Nicht-Zielregion klassifiziert wird, wenn das Durchschnittsverhältnis, oder wenn alle die Verhältnisse, die für die Basislinien der entsprechenden regionalen Aufzeichnung berechnet wurden, größer oder gleich einem ersten Wert ist/sind,
wobei die Region andernfalls als eine richtig oder falsch positive Zielregion klassifiziert wird.

11. Verfahren nach Anspruch 9 oder Anspruch 10, wobei eine Region als eine richtig oder falsch positive prioritäre Zielregion klassifiziert wird, wenn das Durchschnittsverhältnis, oder mindestens ein Verhältnis, das für eine Basislinie der entsprechenden regionalen Aufzeichnung berechnet wurde, kleiner oder gleich einem zweiten Wert, gegebenenfalls kleiner als der erste Wert ist.

12. Verfahren nach der Kombination der Ansprüche 10 und 11, wobei eine Region als eine richtig oder falsch positive sekundäre Zielregion klassifiziert wird, wenn das Durchschnittsverhältnis, oder mindestens ein Verhältnis, das für eine Basislinie der entsprechenden regionalen Aufzeichnung berechnet wurde, zwischen dem zweiten Wert und einem dritten Wert liegt, der zwischen dem ersten und zweiten Wert liegt; und
die Region als eine richtig oder falsch positive tertiäre Zielregion klassifiziert wird, wenn das Durchschnittsverhältnis, oder mindestens ein Verhältnis, das für eine Basislinie der entsprechenden regionalen Aufzeichnung berechnet wurde, zwischen dem dritten Wert und dem ersten Wert liegt.

13. Verfahren nach einem der Ansprüche 9 bis 12, wobei die Klassifizierung jeder der Regionen des Herzens ebenfalls von der Wiederholung der Basislinien mit einem berechneten Verhältnis sich wiederholender Werte innerhalb der entsprechenden regionalen Aufzeichnung abhängig ist;
wobei es sich bei einer Zielregion um eine richtig Positive handelt, wenn die Wiederholung nachgewiesen wird.

14. Verfahren nach einem der Ansprüche 9 bis 12, wobei die Klassifizierung jeder der Regionen des Herzens ebenfalls vom Durchschnitt der Amplitudenwerte der Depolarisationen der entsprechenden regionalen Aufzeichnung abhängig ist;
wobei es sich bei einer Zielregion um eine richtig Positive handelt, wenn der Durchschnitt der Amplitudenwerte kleiner als ein gegebener Schwellenwert ist.

15. Verfahren nach einem der Ansprüche 9 bis 12, wobei die Klassifizierung jeder der Regionen des Herzens ebenfalls von der Wiederholung der Basislinien mit einem berechneten Verhältnis sich wiederholender Werte innerhalb der entsprechenden regionalen Aufzeichnung abhängig ist, und vom Durchschnitt der Amplitudenwerte der Depolarisationen der entsprechenden regionalen Aufzeichnung abhängig ist;
wobei es sich bei einer Zielregion um eine richtig Positive handelt, wenn die Wiederholung nachgewiesen wird, und wenn der Durchschnitt der Amplitudenwerte größer als ein gegebener Schwellenwert ist.

16. Vorrichtung zum Identifizieren von Bereichen des Herzens, die an der Aufrechterhaltung des Vorhofflimmerns beteiligt sein könnten, die einen digitalen Analysator und einen digitalen Kartographen umfasst, wobei der digitale Analysator geeignet ist, um das Verfahren nach einem der Ansprüche 1 bis 15 umzusetzen;
wobei der digitale Kartograph geeignet ist, um eine Karte des Herzens des Patienten zu erzeugen, in der die Klassifizierung der Bereiche des Herzens realisiert ist.

17. Computerprogramm, das eine Folge von Anweisungen umfasst, die, wenn sie von einem Prozessor ausgeführt werden, diesen dazu bringen, die Schritte des Verfahrens nach einem der Ansprüche 1 bis 15 umzusetzen.

18. Nichtflüchtiger und computerlesbarer Speicherträger, der ein Computerprogramm nach Anspruch 17 umfasst.

## Claims

1. A method for identifying areas of the heart of a patient likely to be involved in the perpetuation of atrial fibrillation implemented by a processor, hereinafter target areas, from a plurality of local recordings made on the heart of the patient during a measurement window, each local recording corresponding to an area of the heart of the patient, each local recording comprising at least one local EGM and possibly at least one base line, the method comprising the following steps:
- the reception of a reference cycle of the arrhythmia, the reference cycle being the average duration of the base lines between two local EGMs of a local recording corresponding to a healthy area of the heart of the patient; and
- for each local recording:
• the determination of base lines between two local EGMs;
• the calculation of at least one ratio out of:
∘ an average ratio of the duration of the base line to the reference cycle of the arrhythmia;
∘ a median ratio of the duration of the base line to the reference cycle of the arrhythmia;
∘ for each base line, a ratio of the duration of the base line to the reference cycle of the arrhythmia;
• the classification of the corresponding area of the heart according to its degree of involvement in the perpetuation of the AF, the degree of involvement of the area being a function of the ratio or ratios calculated.

2. The method according to claim 1, in which an area is classified as a non-target area if the average ratio, the median ratio, or all the ratios calculated for the base lines of the corresponding local recording are greater than or equal to a first value,
the area otherwise being classified as a true or false positive target area.

3. The method according to claim 1 or claim 2, in which an area is classified as a true or false positive priority target area if the average ratio, the median ratio, or at least one ratio calculated for a base line of the corresponding local recording is less than or equal to a second value, as the case may be, less than the first value.

4. The method as claimed by the combination of claims 2 and 3, in which an area is classified as a true or false positive secondary target area if the average ratio, the median ratio, or at least one ratio calculated for a base line of the corresponding local recording lies between the second value and a third value lying between the first and second values; and
the area is classified as a true or false positive tertiary target area if the average ratio, the median ratio, or at least one ratio calculated for a base line of the corresponding local recording lies between the third value and the first value.

5. The method according to one of claims 1 to 4, in which the classification of each of the areas of the heart is also a function of the repetition within the corresponding local recording of the base lines with calculated ratio of repetitive values;
a target area being a true positive if the repetition corresponding to the ratio value having led to the classification of the area as being a target area is proven.

6. The method according to one of claims 1 to 4, in which the classification of each of the areas of the heart is also a function of the average of the amplitude values of the depolarizations of the corresponding local recording;
a target area being a true positive if the average of the amplitude values is less than a given threshold value.

7. The method according to one of claims 1 to 4, in which the classification of each of the areas of the heart is also a function of the repetition within the corresponding local recording of the base lines with calculated ratio of repetitive values and a function of the average of the amplitude values of the depolarizations of the corresponding local recording; a target area being a true positive if the repetition is proven and if the average of the amplitude values is greater than a given threshold value.

8. The method according to one of claims 1 to 7, further comprising, for at least some of the local recordings of the heart:
- the obtention of at least one regional recording from a plurality of local recordings corresponding to adjacent areas, the adjacent areas forming a region corresponding to the regional recording, the local recordings not exhibiting any identifiable base line being discarded, so the regional recording comprises at least one regional EGM; and
- for each regional recording:
• the determination of base lines between two regional EGMs;
• the calculation of at least one ratio out of:
∘ an average ratio of the duration of the base line to the reference cycle of the arrhythmia; and
∘ for each base line, a ratio of the duration of the base line to the reference cycle of the arrhythmia;
• the classification of the corresponding region of the heart according to its degree of involvement in the perpetuation of the AF, the degree of involvement of the region being a function of the ratio or ratios calculated;
the other steps of the method being carried out by replacing:
- local recording by regional recording and
- area by region.

9. A method for identifying regions of the heart of a patient likely to be involved in the perpetuation of atrial fibrillation, hereinafter target regions, implemented by a processor, from a plurality of regional recordings made on the heart of the patient during a measurement window, each regional recording corresponding to a region of the heart of the patient combining a plurality of areas, the method comprising the following steps:
- the reception of a reference cycle of the arrhythmia, the reference cycle being the average duration of the base lines between two depolarizations of a local recording corresponding to a healthy area of the heart of the patient; and
- for each regional recording:
• the determination of base lines between the depolarizations of the regional recordings;
• the calculation of at least one ratio out of:
∘ an average ratio of the duration of the base line to the reference cycle of the arrhythmia;
∘ for each base line, a ratio of the duration of the base line to the reference cycle of the arrhythmia;
• the classification of the corresponding region of the heart according to its degree of involvement in the perpetuation of the AF, the degree of involvement of the region being a function of the ratio or ratios calculated.

10. The method according to claim 9, in which a region is classified as a non-target region if the average ratio or if all the ratios calculated for the base lines of the corresponding regional recording is/are greater than or equal to a first value,
the region being otherwise classified as a true or false positive target region.

11. The method according to claim 9 or claim 10, in which a region is classified as a true or false positive priority target region if the average ratio or at least one ratio calculated for a base line of the corresponding regional recording is less than or equal to a second value, as the case may be, less than the first value.

12. The method according to the combination of claims 10 and 11, in which a region is classified as a true or false positive secondary target region if the average ratio or at least one ratio calculated for a base line of the corresponding regional recording lies between the second value and a third value lying between the first and second values; and
the region is classified as a true or false positive tertiary target region if the average ratio or at least one ratio calculated for a base line of the corresponding regional recording lies between the third value and the first value.

13. The method according to one of claims 9 to 12, in which the classification of each of the regions of the heart is also a function of the repetition within the corresponding regional recording of the base lines with calculated ratio of repetitive values;
a target region being a true positive if the repetition is proven.

14. The method according to one of claims 9 to 12, in which the classification of each of the regions of the heart is also a function of the average of the amplitude values of the depolarizations of the corresponding regional recording;
a target region being a true positive if the average of the amplitude values is less than a given threshold value.

15. The method according to one of claims 9 to 12, in which the classification of each of the regions of the heart is also a function of the repetition within the corresponding regional recording of the base lines with calculated ratio of repetitive values and a function of the average of the amplitude values of the depolarizations of the corresponding regional recordings;
a target region being a true positive if the repetition is proven and if the average of the amplitude values is greater than a given threshold value.

16. An apparatus for identifying areas of the heart likely to be involved in the perpetuation of atrial fibrillation, comprising a digital analyzer and a digital mapper,
the digital analyzer being adapted to implement the method according to one of claims 1 to 15;
the digital mapper being adapted to generate a map of the heart of the patient on which the classification of the areas of the heart is formed.

17. A computer program comprising a series of instructions which, when executed by a processor, cause the latter to implement the steps of the method according to one of claims 1 to 15.

18. A non-transient and computer-readable storage medium comprising a computer program according to claim 17.
